(19) Europäisches Patentamt
European Patent Office
Office européen des brevets

(11) Numéro de publication : **0 450 669 B1**

(12) # FASCICULE DE BREVET EUROPEEN

(45) Date de publication du fascicule du brevet : **02.02.94 Bulletin 94/05**

(51) Int. Cl.$^5$ : **A61K 35/78,** A61K 7/48

(21) Numéro de dépôt : **91110927.0**

(22) Date de dépôt : **03.05.89**

(54) **Composition cosmétique ou dermatologique notamment à action amincissante ou anti-cellulitique contenant des extraits de cola sous forme liposomale.**

(30) Priorité : **10.05.88 FR 8806306**

(43) Date de publication de la demande : **09.10.91 Bulletin 91/41**

(60) Numéro de publication de la demande initiale en application de l'article 76 CBE : **0 342 100**

(45) Mention de la délivrance du brevet : **02.02.94 Bulletin 94/05**

(84) Etats contractants désignés : **AT BE CH DE ES GB GR IT LI LU NL SE**

(56) Documents cités :
**FR-A- 2 369 840**
**FR-A- 2 554 344**
**FR-A- 2 586 532**
**GB-A- 2 092 445**
**US-A- 4 684 522**

(73) Titulaire : **PARFUMS CHRISTIAN DIOR**
**33, avenue Hoche**
**F-75008 Paris (FR)**

(72) Inventeur : **Andre, Patrice**
**9, rue Charles Vappereau**
**F-45170 Neuville Aux Bois (FR)**
Inventeur : **Dominice, Jocelyne**
**68 rue de la Bretonnerie**
**F-45000 Orleans (FR)**
Inventeur : **Perrier, Pierre**
**22, rue Jousselin**
**F-45000 Orleans (FR)**
Inventeur : **Redziniak, Gérard**
**101, rue des Fauvettes**
**F-45590 St Cyr en Val (FR)**

(74) Mandataire : **Portal, Gérard et al**
**Cabinet Beau de Loménie 158, rue de l'Université**
**F-75340 Paris Cédex 07 (FR)**

EP 0 450 669 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne essentiellement une composition cosmétique ou dermatologique, notamment à action amincissante ou anti-cellulitique, contenant des extraits de cola sous forme liposomale.

Un nombre assez important de préparations cosmétiques destinées à l'amincissement des parties superficielles du corps humain existent actuellement sur le marché. Elles contiennent souvent des produits d'origine naturelle, en particulier d'origine végétale, tels que : extrait de lierre, caféine, escine, etc. La littérature mentionne par ailleurs un grand nombre de formules amincissantes dont on peut citer à titre d'exemples US-A-4 525 359 Greenway III et al., qui propose d'utiliser comme principe actif des β-stimulants adrénergiques, de préférence théophylline, isoprotérénol, forskoline, et épinephrine. De même, antérieurement, il avait été préconisé dans le document US-A-4 288 433 Koulbanis et al., l'emploi de méthylxanthines, en particulier la caféine, dans la préparation de produits amincissants destinés à l'application locale sur les zones à traiter.

Or, jusqu'à présent, aucune des formulations mises sur le marché pour l'amincissement du corps n'a été reconnue très efficace. C'est pourquoi il est assez souvent recommandé d'utiliser ces formulations en combinaison avec un "programme" ou régime d'amaigrissement. C'est en particulier le cas pour ce document US-A-4 525 359 de sorte que l'on n'est jamais sûr de savoir, en cas d'amincissement constaté, si celui-ci est dû au programme ou régime d'amaigrissement, ou à la formulation amincissante.

Il en résulte qu'un grand nombre de principes actifs pour l'amincissement a été proposé par les formulateurs ou les fournisseurs.

En particulier, dans le document FR-A-2 499 405, on décrit une composition cosmétique amincissante et anti-cellulitique à base d'un extrait de plante contenant des saponines, un extrait d'Arnica Montana L. et un extrait de noix de cola, ainsi que son procédé d'application.

Egalement, dans le document FR-A-2 554 344, on décrit encore une composition cosmétique à action amincissante et anti-cellulitique comprenant de 0,5 à 10 % en poids d'une base purique d'origine naturelle ou synthétique, choisie avantageusement parmi les extraits de café, de thé, de cola et de maté, la caféine et la théophylline, ainsi que leurs dérivés tels que leurs sels et leurs complexes ; 0,5 à 10 % en poids d'un extrait de Hedera Helix et 0,1 à 10 % en poids d'un extrait de Ruscus aculeatus.

De même, la société italienne Inverni Della Beffa propose des extraits de graines de cola qui contiennent en particulier des méthylxanthines comprenant la caféine, la théobromine, la théophylline, etc. et des tanins, pour leurs propriétés d'astringence, d'arômatisation et d'activation de la lipase. Cette société propose deux extraits particuliers, à savoir le cola extrait sec, désigné encore "extrait 14" en raison d'un titre à 14 % en alcaloïde exprimé en caféine dont la proportion d'addition conseillée est de jusqu'à 1 %.

Le deuxième extrait consiste en le cola extrait fluide Lipa, encore dénommé "extrait Lipa" dont le titre en alcaloïde exprimé en caféine est inférieur ou égal à 0,5 %o.

Etant donné que l'extrait Lipa est pratiquement exempt d'alcaloïde, et que la propriété amincissante était jusqu'à présent attribuée aux méthylxanthines, en particulier à la caféine, cet extrait Lipa est exclusivement préconisé pour la préparation de compositions astringentes à raison d'une proportion jusqu'à 5 %.

Les recherches intensives qui ont été effectuées par les présents inventeurs ont permis de découvrir de manière totalement inattendue, en étudiant l'action de ces deux extraits, respectivement l'extrait 14 et l'extrait Lipa, sur l'activité des adipocytes en culture, que ces extraits, lorsqu'ils étaient encapsulés en liposomes, présentaient une activité radicalement supérieure aux extraits libres, c'est-à-dire non encapsulés dans des liposomes.

Ainsi, selon un premier aspect, la présente invention fournit une composition cosmétique ou dermatologique, notamment à activité amincissante ou anti-cellulitique, caractérisée en ce qu'elle comprend des phases lamellaires lipidiques hydratées ou des liposomes contenant des extraits totaux ou partiels de graines de cola.

Selon un mode de réalisation particulier de l'invention, les extraits partiels de graines de cola sont des extraits sensiblement exempts de méthylxanthines ayant une teneur en méthylxanthine inférieure à environ 0,5 %o, tandis que les extraits totaux sont des extraits secs à teneur en méthylxanthine comprise entre environ 10 % et 14 %.

Selon un autre mode de réalisation particulier, le cola à partir duquel les extraits selon l'invention sont obtenus est la plante Cola Nitida ou la plante Cola Vera Shum.

Les procédés d'extraction utilisables selon l'invention sont des procédés connus de l'homme de l'art, utilisant par exemple des solvants organiques ou des mélanges de solvants, tels qu'un mélange hydro-alcoolique.

On peut par exemple obtenir des extraits totaux de graines de cola selon l'invention en s'inspirant du procédé décrit dans le document FR-A-2 586 532 légèrement modifié comme suit.

Les graines ou noix de cola sont broyées et ensuite sont soumises à un traitement d'extraction au moyen d'un mélange méthanol:eau ou éthanol:eau à 40-50 %. L'extrait hydro-alcoolique est recueilli et l'alcool est ensuite éliminé par évaporation.

La solution aqueuse ainsi obtenue est alors séchée par atomisation ou lyophilisation, puis broyée pour obtenir un extrait total stable. Cet extrait total peut être utilisé tel quel comme extrait total selon l'invention.

Selon un autre mode de réalisation particulier de l'invention, les extraits de graines de cola selon l'invention, sensiblement exempts de méthylxanthines, sont obtenus par tout procédé d'extraction capable d'éliminer sensiblement complètement les méthylxanthines.

Ainsi, il est possible, à partir de la solution aqueuse obtenue lors de la mise en oeuvre du procédé décrit ci-dessus, de préparer un extrait sensiblement exempt de méthylxanthines, et en particulier sensiblement exempt de caféine, selon l'invention, par un traitement au moyen d'un solvant sélectif de la caféine, tel qu'un solvant chloré comme le dichlorométhane, le chloroforme ou le trichloroéthane, ou un autre solvant seul ou une combinaison comme indiqué dans le document US-A-4 279 937 ou EP-B-101 135, à savoir : l'alcool benzylique, la méthyléthylcétone ou l'acétate de méthyle.

On peut également utiliser le procédé décrit dans le document US-A-4 279 937 de Procter, applicable à la noix de cola, utilisant un mélange alcool benzylique avec un autre solvant, tel que xylène, acétate d'éthyle, cyclopentane, cyclohexane.

Selon une variante de réalisation, les méthylxanthines, et en particulier la caféine, sont extraites seules, dans un premier temps, à partir des graines de cola, ces graines ayant été de préférence broyées au préalable. On procède ensuite à l'extraction des graines de cola décaféinées selon un procédé classique tel que celui décrit plus haut pour l'obtention de l'extrait total.

Plusieurs procédés d'extraction de caféine, donc de méthylxanthines sont décrits dans la littérature. Par exemple, dans le Journal of the Agricultural Chemical Society of Japan, (1985), <u>59</u>, n° 9, pages 917-919, on décrit un procédé d'extraction sélectif de caféine par l'eau chaude de feuille de thé intacte, qui est utilisable directement pour l'extraction des méthylxanthines dans l'eau. Ce procédé, appliqué aux graines de noix de cola broyées permet d'obtenir des graines de cola décaféinées.

On peut encore éliminer les méthylxanthines en utilisant un procédé d'extraction à l'aide d'un gaz à l'état supercritique, tel que le dioxyde de carbone, comme décrit dans le document Food Technology (chicago) (1986), 40, n° 7, pages 57-64, ainsi que le document Journal of Food Sciences and Technology, (1986), 23, n° 6, pages 326-328.

Dans les compositions selon l'invention, la proportion en poids des extraits de cola, par rapport au poids total de la composition peut varier en de larges limites. Les proportions préférées sont des proportions en poids comprises entre 0,01 et 10 % en poids par rapport au poids total de la composition cosmétique ou dermatologique.

L'incorporation des extraits de graines de cola totaux ou partiels dans des phases lamellaires lipidiques hydratées ou dans des liposomes peut être effectuée selon tout procédé connu. Ceux-ci sont choisis plus particulièrement en fonction des caractères plus ou moins lipophiles ou plus ou moins hydrophiles des extraits à incorporer.

Selon un mode préféré d'incorporation, on choisit une technique de préparation décrite dans le document EP-B1-0087 993, éventuellement en combinaison avec une technique décrite dans le document EP-B1-0107 159.

Ainsi, il est par exemple possible d'inclure les extraits de graines de cola totaux ou partiels dans des phases lamellaires lipidiques hydratées ou des liposomes.

Dans la présente description et les revendications, le terme "lipidique" dans l'exemple "phase lamellaire lipidique" couvre toutes les substances comprenant une chaîne carbonée dite grasse, généralement supérieure à 5 atomes de carbone, cette substance étant habituellement dénommée "lipide".

Selon l'invention, on utilise à titre de lipides pour former soit le liposome précité, soit les phases lamellaires lipidiques, des lipides amphiphiles, c'est-à-dire constitués de molécules possédant un groupe hydrophile indifféremment ionique ou non ionique et un groupe lipophile, ces lipides amphiphiles étant susceptibles de former des phases lamellaires lipidiques en présence d'une phase aqueuse.

En particulier, on peut citer parmi ces lipides : les phospholipides, les phosphoaminolipides, les glycolipides, les alcools gras polyoxyéthylénés, les esters de polyols éventuellement polyoxyéthylénés. De telles substances sont par exemple constituées par une lécithine d'oeuf ou de soja, une phosphatidylsérine, une sphyngomyéline, un cérébroside ou un stéarate de polyglycérol oxyéthyléné. On peut préparer ces phases lamellaires lipidiques hydratées ou les liposomes de la manière suivante :

<u>Etape 1</u>

On dissout dans un solvant organique à point d'ébullition relativement bas, par exemple inférieur à 100°C, à la pression atmosphérique, tel que du dichlorométhane ou du méthanol, un lipide amphiphile, comme par exemple de la lécithine de soja, hydrogénée ou non. On peut, en outre, dissoudre un lipide hydrophobe tel

qu'un stérol, comme le cholestérol ou le β-sitostérol, et avantageusement un antioxydant comme l'α-tocophérol.

La quantité de lipides hydrophobes ne doit généralement pas être supérieure en poids à 0,2 fois la quantité de lipides amphiphile.

Etape 2

Si l'on désire que les extraits totaux ou partiels de graines de cola soient incorporés dans la phase lipidique, on peut dissoudre dans la solution obtenue à l'étape 1, de tels extraits. Dans le cas où une fraction des extraits n'est pas solubilisée, cette fraction insoluble est éliminée par filtration. Les proportions relatives de lipides d'une part et d'extraits d'autre part comme principes actifs peuvent par exemple être comprises, en poids, entre 8 : 2 et 9,9 : 0,1. De préférence, le mélange est agité pendant 30 min à température ordinaire.

Etape 2 bis

Dans le cas où l'on souhaite encapsuler les extraits totaux ou partiels de graines de cola dans la phase aqueuse à l'intérieur des phases lamellaires lipidiques hydratées, au lieu de procéder à l'étape 2 ci-dessus, les extraités précités seront dissous dans l'eau et de préférence dans une solution aqueuse appropriée telle qu'une solution tampon type "tampon phosphate PBS".

Etape 3-A

Le mélange obtenu à la fin de l'étape 1 ou de l'étape 2 est introduit dans un ballon rotatif, et évaporé par chauffage au bain-marie, éventuellement sous pression réduite.

Après évaporation du solvant organique, le film lipidique déposé sur les parois est repris sous agitation par de l'eau ou par une solution aqueuse appropriée telle qu'une solution tampon type "tampon phosphate PBS".

Lorsque l'on utilise le mélange directement obtenu à l'étape 1, sans passer par l'étape 2, on utilise la solution aqueuse obtenue à la fin de l'étape 2 bis.

De préférence, la quantité d'eau ou de solution aqueuse est au moins égale, en poids, à 8 fois la quantité de lipides contenue dans le ballon.

On obtient ainsi une suspension de liposomes, qui peut être ensuite homogénéisée par un moyen approprié, comme par exemple les ultrasons.

Etape 3-B

Selon une variante du procédé de préparation des compositions de l'invention contenant les extraits précités incorporés dans des phases lamellaires lipidiques hydratées ou des liposomes, on utilise le procédé décrit dans le document EP-B1-0087 993, comprenant l'atomisation du mélange obtenu à la fin de l'étape 1 ou de l'étape 2, suivi de la dispersion de la poudre lipidique ainsi obtenue dans une quantité prédéterminée d'eau ou d'une solution aqueuse de substances à encapsuler, en particulier celle obtenue à l'étape 2 bis.

On obtient ainsi des phases lamellaires lipidiques peu hydratées ou une suspension de liposomes, selon que l'on a choisi de disperser la poudre lipidique dans peu ou beaucoup de milieu aqueux, ainsi qu'il est exposé dans le document européen précité.

La dispersion de phases lamellaires hydratées ou des liposomes peut ensuite être homogénéisée, par exemple selon le procédé décrit dans le document EP-B1-0 107 559.

Etape 4 éventuelle

Eventuellement, la dispersion des phases lamellaires lipidiques hydratées ou les suspensions de liposomes obtenues à l'étape 3-A ou 3-B ci-dessus, peuvent être gélifiées, par exemple en les mélangeant avec un gel préparé séparément, comme un gel de polymère vinylique.

D'autres buts, caractéristiques et avantages de l'invention apparaîtront clairement à la lumière de la description explicative qui va suivre faite en référence à plusieurs exemples illustratifs qui ne sauraient en aucune façon limiter la portée de l'invention. Dans ces exemples, les pourcentages sont donnés en poids, sauf indication contraire.

Exemple 1

Préparation d'une composition simple à base d'extraits de graines de cola

On utilise comme matière première de départ, les extraits de graines de cola commercialisés par la société italienne Inverni Della Beffa sous la dénomination cola extrait fluide Lipa ayant une teneur inférieure ou égale à 0,5 %o en alcaloïdes exprimée en caféine, ci-après dénommée cola Lipa.

A partir de cet extrait, on prépare des compositions selon l'invention ayant différentes proportions de cola Lipa à 0,1 %, 0,5 % et 2 % par dissolution de cet extrait dans une solution aqueuse tampon phosphate ou PBS. Après dissolution, on filtre la solution obtenue sur filtre à 0,8 μm pour supprimer la substance insoluble.

On obtient ainsi des compositions selon l'invention qui peuvent être utilisées telles quelles et notamment pour les essais d'activité qui seront décrits plus loin.

A titre de comparaison, on prépare également des compositions comparatives à base du produit commercialisé par la même société italienne Inverni, dénommé cola extrait sec et ayant une teneur égale à environ 14 % en alcaloïdes exprimée en caféine, ci-après dénommé cola 14.

On procède de la même manière avec cet extrait cola 14 pour préparer des solutions à 0,1 %, 0,5 % et 2 %.

Exemple 2

Composition liposomale selon l'invention contenant un extrait de graines de cola sensiblement exempt de méthylxanthine

Pour ce faire, on peut préparer également diverses compositions ayant diverses teneurs d'extraits à 0,1 %, 0,5 % et 2 %, sous forme liposomale, de la manière suivante :

on peut partir des solutions contenant du cola Lipa en solution aqueuse préparée à l'exemple 1 auxquelles on ajoute 1 % de poudre lipidique obtenue par le procédé d'atomisation décrit dans le document EP-B1-0 087 993, décrit succintement à l'étape 3-B précitée, et qui comprend 9 parties de lécithine de soja naturelle hydrogénée pour 1 partie de β-sitostérol.

On disperse la poudre lipidique sous agitation magnétique dans la solution aqueuse jusqu'à obtention d'une suspension à l'état homogène.

On procède ensuite à une ultrasonication de ces solutions, par exemple avec un appareil d'ultrasonication dénommé LABSONIC 1510, pendant 7 min à 200 W et à 4°C, afin d'obtenir des liposomes dont la taille est comprise entre 100 et 150 nm tel que déterminé au Nanosizer® (Coultronic).

Par cette procédure, on obtient ainsi des compositions liposomales contenant respectivement 0,1 %, 0,5 % et 2 % en poids de cola Lipa, conformément à l'invention, qui peuvent être utilisées telles quelles pour constituer des compositions cosmétiques ou dermatologiques, ou en combinaison avec d'autres agents actifs et/ou excipients comme cela sera exemplifié plus loin. Ces compositions sont également utilisées plus loin pour tester leur activité.

Exemple 3

Composition liposomale contenant des extraits totaux de graines de cola

On procède comme à l'exemple 2 si ce n'est que l'on utilise au départ les solutions aqueuses préparées à l'exemple 1-B contenant de l'extrait de cola 14 en lieu et place du cola Lipa.

On obtient ainsi des compositions liposomales respectivement à 0,1 %, 0,5 % et 2 % de cola 14. Ces compositions peuvent également être utilisées telles quelles pour la préparation de composition cosmétique ou pharmaceutique, notamment dermatologique, ou être complétées par d'autres agents actifs et/ou des excipients, comme cela sera décrit plus loin. Ces compositions sont en outre utilisées telles quelles pour tester leur activité.

Exemple 4

Mise en évidence de l'activité lipolytique des compositions selon l'invention

- Evaluation de l'action des compositions selon l'invention sur les adipocytes en culture.

Il a été choisi d'évaluer l'efficacité des compositions selon l'invention en tant qu'agents lipolytiques sur une lignée de pré-adipocytes murins, telle qu'une lignée 3T3 L1 disponible dans le commerce auprès de la Société Flow Laboratories, qui ont été sélectionnés pour leur capacité à se convertir en adipocytes si les conditions de culture le permettent.

(Conformément à la méthode de Green, H & Kehinde, C, Cell 1 (1974) 113).

Cette lignée constitue en effet un modèle d'étude de la différenciation adipocytaire in vitro tout en offrant la possibilité lorsque le phénotype adipocytaire est atteint d'étudier les régulations du fonctionnement cellulaire selon l'environnement extra-cellulaire. Cette différenciation et sa modulation s'accompagnent d'un certain nombre de modifications morphologiques et biochimiques et en particulier, les modifications biochimiques concernent la libération du glycérol.

Il est donc commode de vérifier l'efficacité lipolytique des composés à tester par un dosage du glycérol libéré dans le milieu de culture après plusieurs jours de traitement.

Ces expérimentations sont réalisées comme suit :

1) Conditions de culture

Les pré-adipocytes sont ensemencés dans des boîtes de Pétri de 35 mm de diamètre (20 000 cellules/boîte), en présence de DMEM ("Dulbecco's Modified Eagle Medium") contenant 10 % de sérum de veau foetal et des antibiotiques (pénicilline, streptomycine).

Le milieu est renouvelé tous les 2-3 jours.

Dans ces conditions, la culture atteint la confluence en une semaine (J = Jo), à ce stade, la différenciation adipocytaire est activée par l'addition d'insuline à la concentration de 5 µg/ml de milieu de culture.

Ces cellules présentent un état différencié avancé une semaine après la confluence ($J = J_7$).

2) Traitement - Viabilité

Les cellules sont traitées par les produits à tester au stade $J_7$.

Ce traitement consiste à remplacer le milieu de culture soit par du milieu complet pour le témoin, soit par ce même milieu contenant le produit à tester à différentes concentrations.

Pour le dosage du glycérol libéré, le milieu de culture est récupéré de 24 h à 7 jours après le traitement.

Le milieu contenant ou non le produit à tester est renouvelé tous les 2-3 jours.

On s'assure de la non toxicité des produits sur les cellules en culture en déterminant le taux de protéine totale selon la méthode de Bradford décrite par M.M. Bradford (Analytical Biochemistry (1976), 72, 248-54, et qui consiste à réaliser une digestion à la soude 0,5 N, et une coloration au bleu de Coomassie. On mesure alors l'importance de la lipolyse en effectuant un dosage par bioluminescence du glycérol libéré dans le milieu de culture.

3) Dosage par bioluminescence du glycérol libéré dans le milieu de culture

Les milieux de culture des témoins ou des traités sont récupérés 2 jours à 7 jours après le traitement.

Les surnageants peuvent être stockés à -20°C avant dosage.

La méthode de dosage du glycérol est la suivante :

On dose le glycérol par la réaction de celui-ci avec l'ATP en présence de l'enzyme glycérolkinase selon la réaction suivante :

glycérol + ATP glycérolkinase glycérol 3 phosphate + ADP.

L'excès d'ATP est dosé instantanément par bioluminescence, dès que celui-ci est mis en présence de complexe luciférine-luciférase, selon la réaction suivante :

$$\text{luciférine} + \text{luciférase} + \text{ATP} \xrightarrow{Mg^{2+}} \text{luciférine-luciférase-AMP} + \text{pyrophosphate}$$

luciférine-luciférase-AMP $\xrightarrow{O_2}$ oxyluciférine + luciférase + AMP + $CO_2$ + émission lumineuse.

L'émission lumineuse est captée par un photomultiplicateur, par exemple un biocounter Lumac® 3 M.

Lors du mélange des réactifs, le maximum d'émission lumineuse est instantané. Le dosage proprement dit a lieu de la manière suivante :

a) <u>Préparation de l'ATP</u>

On prépare une solution d'ATP en provenance de chez Sigma à 50 μM dans une solution de $MgSO_4$, $7H_2O$ à 0,2 mM.

L'ATP est congelé à -20°C et lors de l'utilisation il subira une décongélation douce et sera conservé à 0°C (dans la glace) et à l'obscurité.

b) <u>Préparation des échantillons</u>

On prépare une gamme d'étalonnage de glycérol à 0,25, 0,50, 0,75, 1 mM dans le tampon acide citrique/NaOH 0,1 M à pH 7.

Le dosage du glycérol présent dans le milieu de culture s'effectue après congélation et décongélation douce du milieu. On choisit la dilution afin de se trouver entre les concentrations de 0,20 à 0,90 nanomoles par ml de volume réactionnel.

On effectue 3 essais par échantillon.

c) <u>Méthode de dosage</u>

On met dans une cuve de mesure 10 μl d'ATP 50 μM puis 20 μl de glycérolkinase disponible chez Boehringer, Mannheim, de préférence purifiée et 770 μl de tampon acide citrique/NaOH 0,1 M à pH 7.

On ajoute 100 μl de solution de glycérol (gamme étalonnage) ou de milieu de culture convenablement dilué.

On agite vigoureusement pendant 10 s.

On incube 4 min à 20-22°C à l'obscurité.

On injecte 100 μl de luciférine-luciférase (NRB/lumi PM Kit de chez Lumac 3 M) à l'aide de la pompe de biocounter Lumac 3 M.

On intègre sur 60 s.

On fait trois essais par échantillon.

d) <u>Expression mathématique des résultats</u>

Entre la lumière émise y et la concentration x en glycérol, on opère par régression suivant la méthode des moindres carrés. On se base sur deux critères pour sélectionner l'équation : le coefficient de détermination $R^2$ et l'erreur standard de la régression (équivalent du bruit de fond de l'expérience).

La forme polynomiale traduit le mieux le phénomène. Seuls les deux premiers degrés apportent une réelle information.

Dans l'équation, on ne retient que l'équation du deuxième degré représentant la variation de y de lumière émise en fonction de la concentration x en glycérol de la forme suivante :

$$y = a - bx + cx^2$$

Le coefficient de détermination de la courbe d'étalonnage doit être supérieur à 90 % pour considérer la gamme comme étant valable.

Le coefficient de variation pour chacune des concentrations de la gamme d'étalonnage obtenue par bioluminescence est inférieur à 5 %.

e) <u>Résultats</u>

On soumet à l'essai le dosage par bioluminescence du glycérol dans le milieu de culture après 2 jours et 7 jours de traitement par les extraits de cola 14 et de cola Lipa à une concentration de 1 mg/l à l'état libre comme décrit à l'exemple 1 et sous forme liposomale comme décrit aux exemples 2 et 3.

On réalise trois essais par échantillon prélevé sur trois cultures réalisées dans les mêmes conditions. On obtient les résultats suivants :

## RESULTATS

Après 2 jours de traitement

| | Actifs seuls | | | Actifs en liposomes | | |
|---|---|---|---|---|---|---|
| | Témoin | cola 14 % | cola Lipa | Liposome blanc (témoin) | Liposome cola 14 % | Liposome cola Lipa |
| µ mol de glycérol/mL | 0,79 | 1,34 | 1,62 | 1,80 | 2,70 | 3,90 |
| de milieu de | 0,59 | 1,19 | 0,81 | 1,04 | 3,80 | 6,37 |
| culture | 0,96 | 0,71 | 1,25 | 0,52 | 0,90 | 1,24 |
| moyenne en µ mol/ml | 0,78 | 1,08 | 1,23 | 1,12 | 2,46 | 3,83 |
| | 0,19 | 0,33 | 0,41 | 0,64 | 1,46 | 2,57 |
| % relatif en glycérol | 100 | 138 | 158 | 100 | 220 | 342 |

Après 7 jours de traitement

| | Actifs seuls | | | Actifs en liposomes | | |
|---|---|---|---|---|---|---|
| | Témoin | cola 14 % | cola Lipa | Liposome blanc (témoin) | Liposome cola 14 % | Liposome cola Lipa |
| µ mol de glycérol/mL | 1,36 | 1,48 | 1,20 | 1,76 | 3,45 | 4,20 |
| de milieu de | 0,88 | 1,18 | 1,34 | 1,65 | 4,80 | 5,90 |
| culture | 0,75 | 0,94 | 1,48 | 0,76 | 4,20 | 4,90 |
| moyenne en µ mol/ml | 1,00 | 1,20 | 1,34 | 1,39 | 4,15 | 5,00 |
| | 0,32 | 0,27 | 0,14 | 0,55 | 0,68 | 0,85 |
| % relatif en glycérol | 100 | 120 | 134 | 100 | 299 | 360 |

On peut ainsi constater, de manière totalement inattendue, que les extraits de cola Lipa ou de cola 14 libres, c'est-à-dire non incorporés dans des liposomes, ont une activité lipolytique relativement faible, tandis que sous forme liposomale, cette activité est radicalement accrue, cette augmentation d'activité étant encore beaucoup plus significative et radicale pour l'extrait cola Lipa sensiblement exempt de méthylxanthines, ce qui est totalement surprenant, même par rapport à la forme non liposomale.

Divers exemples de formulations de compositions cosmétiques ou pharmaceutiques, notamment dermatologiques, sont maintenant décrits.

Exemple 5

Gel dermatologique amincissant aux liposomes

On prépare une suspension de liposomes selon l'exemple 2, encapsulant l'extrait de cola fluide Lipa. Cette suspension est ensuite mélangée à un gel de Carbopol 940® neutralisé préparé séparément.

On obtient ainsi la composition gélifiée suivante :

| | |
|---|---|
| Lécithine de soja | 1,0 g |
| β-sitostérol | 0,1 g |
| Extrait de cola fluide Lipa | 1,0 g |
| Carbopol 940® | 0,4 g |
| Excipients aqueux stabilisés par des conservateurs et antioxydants | qsp 100 g |

Cette préparation, appliquée quotidiennement à la taille, aux cuisses et aux hanches permet d'obtenir une diminution sensible de la cellulite en l'espace d'une à trois semaines.

Exemple 6

Emulsion amincissante aux liposomes

Composition :

| | |
|---|---|
| Lécithine de soja | 1,5 g |
| Extrait de cola 14 | 2,0 g |
| Squalane | 8,5 g |
| Conservateurs | 0,15 g |
| Excipients aqueux gélifiés et parfumés | 100,0 g |

Pour obtenir cette composition, on réalise d'abord une suspension de liposomes selon la méthode de l'exemple 3, encapsulant l'extrait de cola 14. Cette suspension est ensuite gélifiée par un gel de Carbopol 940® neutralisé à 1,25 % préparé séparément.

Le gel obtenu est émulsionné avec une phase huileuse à base de squalane.

L'émulsion obtenue, de consistance agréable peut être appliquée quotidiennement sur les parties du corps à traiter par cure de deux à trois semaines.

## Revendications

### Revendications pour les Etats contractants suivants : AT, BE, CH, DE, GB, IT, LI, LU, NL, SE

1. Composition cosmétique ou dermatologique, notamment à activité amincissante ou anti-cellulitique, caractérisée en ce qu'elle comprend des phases lamellaires lipidiques hydratées ou des liposomes contenant des extraits totaux ou partiels de graines de cola, contenant des méthylxanthines.

2. Composition selon la revendication 1, caractérisée en ce que les extraits totaux sont des extraits secs à teneur en méthylxanthine comprise entre environ 10 et environ 14 % en poids.

3. Composition selon la revendication 1 ou 2, caractérisée en ce que le cola à partir duquel les extraits selon l'invention sont obtenus est la plante Cola Nitida ou la plante Cola Vera Schum.

4. Composition selon l'une des revendications 1 à 3, caractérisée en ce que la proportion en poids de l'extrait est comprise entre 0,01 et 10 % en poids par rapport au poids total de la composition.

5. Utilisation de phases lamellaires lipidiques hydratatées ou de liposomes contenant des extraits totaux partiels de graines de cola contenant des méthyxanthines pour la préparation d'une composition cosmétique ou dermatologique à activité amincissante ou anti-cellulitique.

6. Utilisation selon la revendication 5, caractérisée en ce que la proportion en poids de l'extrait est comprise entre 0,01 et 10 % en poids par rapport au poids total de la composition.

**Revendications pour l'Etat contractant suivant : GR**

1. Composition cosmétique, notamment à activité amincissante ou anti-cellulitique, caractérisée en ce qu'elle comprend des phases lamellaires lipidiques hydratées ou des liposomes contenant des extraits totaux ou partiels de graines de cola, contenant des méthylxanthines.

2. Composition selon la revendication 1, caractérisée en ce que les extraits totaux sont des extraits secs à teneur en méthylxanthine comprise entre environ 10 et environ 14 % en poids.

3. Composition selon l'une des revendications 1 et 2, caractérisée en ce que le cola à partir duquel les extraits selon l'invention sont obtenus est la plante Cola Nitida ou la plante Cola Vera Schum.

4. Composition selon l'une des revendications 1 à 3, caractérisée en ce que la proportion en poids de l'extrait est comprise entre 0,01 et 10 % en poids par rapport au poids total de la composition.

5. Utilisation de phases lamellaires lipidiques hydratées ou de liposomes contenant des extraits totaux ou partiels de graines de cola, pour la préparation d'une composition cosmétique ou dermatologique, notamment à activité amincissante ou anti-cellulitique.

6. Procédé de préparation d'une composition cosmétique ou dermatologique, notamment à activité amincissante ou anti-cellulitique, caractérisé en ce qu'il comprend l'incorporation de phases lamellaires lipidiques hydratées ou de liposomes contenant des extraits totaux ou partiels de graines de cola, contenant des méthylxanthines, dans un excipient cosmétiquement ou dermatologiquement acceptable.

7. Procédé selon la revendication 6, caractérisé en ce que les extraits totaux sont des extraits secs à teneur en méthylxanthine comprise entre environ 10 et environ 14 % en poids.

8. Procédé selon l'une des revendications 6 et 7, caractérisé en ce que le cola à partir duquel les extraits selon l'invention sont obtenus est la plante Cola Nitida ou la plante Cola Vera Schum.

9. Procédé selon l'une des revendications 6 à 8, caractérisé en ce que la proportion en poids de l'extrait est comprise entre 0,01 et 10 % en poids par rapport au poids total de la composition.

**Revendications pour l'Etat contractant suivant : ES**

1. Utilisation de phases lamellaires lipidiques hydratées ou de liposomes contenant des extraits totaux ou partiels de graines de cola, contenant des méthylxanthines, pour la préparation d'une composition cosmétique ou dermatologique, notamment à activité amincissante ou anti-cellulitique.

2. Procédé de préparation d'une composition cosmétique ou dermatologique, notamment à activé amincissante ou anti-cellulitique, caractérisé en ce qu'il comprend l'incorporation de phases lamellaires lipidiques hydratées ou de liposomes contenant des extraits totaux ou partiels de graines de cola, contenant des méthylxanthines, dans un excipient cosmétiquement ou dermatologiquement acceptable.

3. Procédé selon la revendication 2, caractérisé en ce que les extraits totaux sont des extraits secs à teneur en méthylxanthine comprise entre environ 10 et environ 14 % en poids.

4. Procédé selon l'une des revendications 2 et 3, caractérisé en ce que le cola à partir duquel les extraits selon l'invention sont obtenus est la plante Cola Nitida ou la plante Cola Vera Schum.

5. Procédé selon l'une des revendications 2 à 4, caractérisé en ce que la proportion en poids de l'extrait est comprise entre 0,01 et 10 % en poids par rapport au poids total de la composition.

**Patentansprüche**

**Patentansprüche für folgende Vertragsstaaten : AT, BE, CH, DE, GB, IT, LI, LU, NL, SE**

1. Kosmetische oder dermatologische Zusammensetzung, insbesondere mit schlankmachender oder zellulitishemmender Aktivität, dadurch gekennzeichnet, daß sie wasserhaltige lamellare Lipidphasen oder Liposome aufweist, die methylxanthinhältige Voll- oder Teilextraxte von Colakörnern enthalten.

2. Zusammensetzung nach Anspruch 1, dadurch gekennzeichnet, daß die Vollextrakte Trockenextrakte mit einem Methylxanthingehalt zwischen etwa 10 und etwa 14 Gew.% sind.

3. Zusammensetzung nach Anspruch 1 oder 2, dadurch gekennzeichnet, daß die Cola, aus der die erfindungsgemäßen Extrake gewonnen werden, die Pflanze Cola Nitida oder die Pflanze Cola Vera Schum ist.

4. Zusammensetzung nach einem der Ansprüche 1 bis 3, dadurch gekennzeichnet, daß das Gewichtsverhältnis des Extraktes zwischen 0,01 und 10 Gew.%, bezogen auf das Gesamtgewicht der Zusammensetzung, beträgt.

5. Verwendung von wasserhaltigen lamellaren Lipidphasen oder von Liposomen, die methylxanthinhältige Voll- oder Teilextrakte von Colakörnern enthalten, zur Herstellung einer kosmetischen oder dermatologischen Zusammensetzung mit schlankmachender oder zellulitishemmender Aktivität.

6. Verwendung nach Anspruch 5, dadurch gekennzeichnet, daß das Gewichtsverhältnis des Extraktes zwischen 0,01 und 10 Gew.%, bezogen auf das Gesamtgewicht der Zusammensetzung, beträgt.

**Patentansprüche für folgenden Vertragsstaat : GR**

1. Kosmetische Zusammensetzung, insbesondere mit schlankmachender oder zellulitishemmender Aktivität, dadurch gekennzeichnet, daß sie wasserhaltige lamellare Lipidphasen oder Liposome aufweist, die methylxanthinhältige Voll- oder Teilextraxte von Colakörnern enthalten.

2. Zusammensetzung nach Anspruch 1, dadurch gekennzeichnet, daß die Vollextrakte Trockenextrakte mit einem Methylxanthingehalt zwischen etwa 10 und etwa 14 Gew.% sind.

3. Zusammensetzung nach Anspruch 1 oder 2, dadurch gekennzeichnet, daß die Cola, aus der die erfindungsgemäßen Extrake gewonnen werden, die Pflanze Cola Nitida oder die Pflanze Cola Vera Schum ist.

4. Zusammensetzung nach einem der Ansprüche 1 bis 3, dadurch gekennzeichnet, daß das Gewichtsverhältnis des Extraktes zwischen 0,01 und 10 Gew.%, bezogen auf das Gesamtgewicht der Zusammensetzung, beträgt.

5. Verwendung von wasserhaltigen lamellaren Lipidphasen oder von Liposomen, die Voll- oder Teilextrakte von Colakörnern enthalten, zur Herstellung einer kosmetischen oder dermatologischen Zusammensetzung, insbesondere mit schlankmachender oder zellulitishemmender Aktivität.

6. Verfahren zur Herstellung einer kosmetischen oder dermatologischen Zusammensetzung, insbesondere mit schlankmachender oder zellulitishemmender Aktivität, dadurch gekennzeichnet, daß es die Einarbeitung von wasserhaltigen lamellaren Lipidphasen oder von Liposomen, die methylxanthinhältige Voll- oder Teilextrakte von Colakörnern enthalten, in einen kosmetisch oder dermatologisch akzeptablen Exzipienten umfaßt.

7. Verfahren nach Anspruch 6, dadurch gekennzeichnet, daß die Vollextrakte Trockenextrakte mit einem Methylxanthingehalt zwischen etwa 10 und etwa 14 Gew.% sind.

8. Verfahren nach einem der Ansprüche 6 und 7, dadurch gekennzeichnet, daß die Cola, aus der die erfindungsgemäßen Extrake gewonnen werden, die Pflanze Cola Nitida oder die Pflanze Cola Vera Schum ist.

9. Verfahren nach einem der Ansprüche 6 bis 8, dadurch gekennzeichnet, daß das Gewichtsverhältnis des Extraktes zwischen 0,01 und 10 Gew.%, bezogen auf das Gesamtgewicht der Zusammensetzung, beträgt.

**Patentansprüche für folgenden Vertragsstaat : ES**

1. Verwendung von wasserhaltigen lamellaren Lipidphasen oder von Liposomen, die methylxanthinhältige Voll- oder Teilextrakte von Colakörnern enthalten, zur Herstellung einer kosmetischen oder dermatologischen Zusammensetzung, insbesondere mit schlankmachender oder zellulitishemmender Aktivität.

2. Verfahren zur Herstellung einer kosmetischen oder dermatologischen Zusammensetzung, insbesondere mit schlankmachender oder zellulitishemmender Aktivität, dadurch gekennzeichnet, daß es die Einarbeitung von wasserhaltigen lamellaren Lipidphasen oder von Liposomen, die methylxanthinhältige Voll- oder Teilextrakte von Colakörnern enthalten, in einen kosmetisch oder dermatologisch akzeptablen Exzipienten umfaßt.

3. Verfahren nach Anspruch 2, dadurch gekennzeichnet, daß die Vollextrakte Trockenextrakte mit einem Methylxanthingehalt zwischen etwa 10 und etwa 14 Gew.% sind.

4. Verfahren nach einem der Ansprüche 2 und 3, dadurch gekennzeichnet, daß die Cola, aus der die erfindungsgemäßen Extrake gewonnen werden, die Pflanze Cola Nitida oder die Pflanze Cola Vera Schum ist.

5. Verfahren nach einem der Ansprüche 2 bis 4, dadurch gekennzeichnet, daß das Gewichtsverhältnis des Extraktes zwischen 0,01 und 10 Gew.%, bezogen auf das Gesamtgewicht der Zusammensetzung, beträgt.

**Claims**

**Claims for the following Contracting States : AT, BE, CH, DE, GB, IT, LI, LU, NL, SE**

1. Cosmetic or dermatological composition, in particular with slimming or anti-cellulitis activity, characterized in that it comprises hydrated lipidic lamellar phases or liposomes containing total or partial extracts of kola, containing methylxanthines.

2. Composition according to claim 1, characterized in that the total extracts are dry extracts with a methylxanthine content comprised between about 10 and about 14% by weight.

3. Composition according to claim 1 or 2, characterized in that the kola from which the extracts according to the invention are obtained is the Kola Nitida plant or the kola Vera Schum plant.

4. Composition according to one of claims 1 to 3, characterized in that the proportion by weight of the extract is between 0.01 and 10% by weight with respect to the total weight of the composition.

5. Use of hydrated lipidic lamellar phases or of liposomes containing total or partial extracts of kola seeds containing methylxanthines for the preparation of a cosmetic or dermatological composition with slimming or anti-cellulitis activity.

6. Use according to claim 5, characterized in that the proportion by weight of the extract is between 0.01 and 10% by weight with respect to the total weight of the composition.

**Claims for the following Contracting State : GR**

1. Cosmetic composition, in particular with slimming or anticellulitis activity, characterized in that it comprises hydrated lipidic lamellar phases or liposomes containing total or partial extracts of kola seeds, containing methylxanthines.

2. Composition according to claim 1, characterized in that the total extracts are dry extracts with a methylxanthine content comprised between about 10 and about 14% by weight.

3. Composition according to one of claims 1 and 2, characterized in that the kola from which the extracts according to the invention are obtained is the Kola Nitida plant or the kola Vera Schum plant.

4. Composition according to one of claims 1 to 3, characterized in that the proportion by weight of the extract is between 0.01 and 10% by weight with respect to the total weight of the composition.

5. Use of hydrated lipidic lamellar phases or of liposomes containing total or partial extracts of kola seeds, for the preparation of a cosmetic or dermatological composition, in particular with slimming or anticellulitis activity.

6. Method for the preparation of a cosmetic or dermatological composition, in particular with slimming or anti-cellulitis activity, characterized in that it comprises the incorporation of hydrated lipidic lamellar phases or of liposomes containing total or partial extracts of kola seeds, containing methylxanthines, in a cosmetically or dermatologically acceptable excipient.

7. Method according to claim 6, characterized in that the total extracts are dry extracts with a methylxanthine content comprised between about 10 and about 14% by weight.

8. Method according to one of claims 6 and 7, characterized in that the kola from which the extracts according to the invention are obtained is the Kola Nitida plant or the kola Vera Schum plant.

9. Method according to one of claims 6 to 8, characterized in that the proportion by weight of the extract is between 0.01 and 10% by weight with respect to the total weight of the composition.

**Claims for the following Contracting State : ES**

1. Use of hydrated lipidic lamellar phases or of liposomes containing total or partial extracts of kola seeds, containing methylxanthines, for the preparation of a cosmetic or dermatological composition, in particular with slimming or anti-cellulitis activity.

2. Method for the preparation of a cosmetic or dermatological composition, in particular with slimming or anti-cellulitis activity, characterized in that it comprises the incorporation of hydrated lipidic lamellar phases or of liposomes containing total or partial extracts of kola seeds, containing methylxanthines, in a cosmetically or dermatologically acceptable excipient.

3. Method according to claim 2, characterized in that the total extracts are dry extracts with a methylxanthine content comprised between about 10 and about 14% by weight.

4. Method according to one of claims 2 and 3, characterized in that the kola from which the extracts according to the invention are obtained is the Kola Nitida plant or the kola Vera Schum plant.

5. Method according to one of claims 2 to 4, characterized in that the proportion by weight of the extract is between 0.01 and 10% by weight with respect to the total weight of the composition.